(19) Europäisches Patentamt
European Patent Office
Office européen des brevets

(11) **EP 1 123 655 B1**

(12) **EUROPEAN PATENT SPECIFICATION**

(45) Date of publication and mention
of the grant of the patent:
**05.10.2005 Bulletin 2005/40**

(51) Int Cl.⁷: $A01N\ 37/16$, $A01N\ 59/26$, $A01N\ 25/30$

(21) Application number: **98947930.8**

(22) Date of filing: **19.10.1998**

(86) International application number:
**PCT/JP1998/004713**

(87) International publication number:
**WO 2000/022931 (27.04.2000 Gazette 2000/17)**

(54) **BACTERICIDAL/DISINFECTANT PERACETIC ACID COMPOSITION**

BAKTERIZIDE DESINFIZIERENDE PERESSIGSÄURE-ZUSAMMENSETZUNG

COMPOSITION BACTERICIDE/DESINFECTANTE A BASE D'ACIDE PERACETIQUE

(84) Designated Contracting States:
**AT BE CH CY DE DK ES FI FR GB GR IE IT LI LU MC NL PT SE**

(43) Date of publication of application:
**16.08.2001 Bulletin 2001/33**

(73) Proprietor: **Saraya Co., Ltd.**
**Osaka 546-0013 (JP)**

(72) Inventors:
• **YASUHARA, Toru Saraya Co., Ltd.**
**Kashiwara-shi Osaka 582-0028 (JP)**
• **YOSHIDA, Emiko Saraya Co., Ltd.**
**Kashiwara-shi Osaka 582-0028 (JP)**

(74) Representative: **Harding, Charles Thomas**
**D Young & Co**
**120 Holborn**
**London EC1N 2DY (GB)**

(56) References cited:
EP-A- 0 131 976        EP-A- 0 873 687
GB-A- 861 660          JP-A- 6 501 913
JP-A- 7 502 988        JP-A- 8 311 495
US-A- 5 545 374        US-A- 5 656 302
US-A- 5 720 983

**Description**

TECHNICAL FIELD

The present invention relates to a field of decontamination. More particularly, the present invention relates to a bactericide/disinfectant for use in medical instruments, and medical equipment such as an endoscope, linen, or other goods.

BACKGROUND ART

At the present time, a preparation including glutaraldehyde as an active component is typically used to sterilize and disinfect medical instruments. However, it has been recently reported that the bactericidal action of the glutaraldehyde preparation is weak against acid-fast bacteria. Further, the glutaraldehyde preparation often causes allergic reactions in Western countries. Therefore, preparations without glutaraldehyde have been increasingly developed and sold.

Whereas a high-concentration peracetic acid causes much irritation and smells badly, the irritation and smell thereof are relatively weak when it is diluted to provide a practical solution (typically having a peracetic-acid concentration of 0.2 to 0.35%). Decomposition products of peracetic acid are acetic acid, water, and hydrogen peroxide (further decomposed to oxygen and water) which are not only harmless but does not cause environment pollution. Therefore, a bactericide/disinfectant including peracetic acid as an active component can be substituted for the glutaraldehyde preparation.

Peracetic acid is typically provided in the form of a peracetic acid preparation and is diluted before use. Peracetic acid is not used to disinfect medical devices in Japan. According to examples in foreign countries, peracetic acid is typically prepared to a final concentration of 0.2 w/v% to 0.35 w/v% before use.

U.S. Patent No. 5,077,008 and Japanese Publication for Opposition No. 7-84362 disclose single use antibacterial compositions which are diluted before use to a final concentration of 0.2% weight per volume (w/v) using a specialized device STERIS SYSTEM 1® (manufactured by Steris Corporation). Nu-Cidex (Johnson & Johnson Medical Inc.) which is commercially available in United Kingdom consists of a peracetic acid thick solution, a stabilizer, and a buffer. The peracetic acid thick solution is diluted with the stabilizer and the buffer to 0.35 w/v% before use. The expiration date for use of the diluted practical solution is 1 to 3 days after the preparation.

There are known peracetic acid preparations including various additives.

Japanese Laid-open Publication No. 8-311495 discloses a peracetic acid preparation including a peracetic acid aqueous solution and a polyether type or the like of nonionic surfactant. The peracetic acid preparation is used as a bleach and disinfectant for a dishwasher or the like. The nonionic surfactant is used as a "rinsing agent" after washing to prevent "spots" from remaining on the surfaces of dishes or the like.

International Publication No. WO 88/08667 discloses peracetic-acid-containing microbicides which are stable and shippable. The microbicides include surfactants, such as sorbitan monopalmitate and polyoxyethylene(2) cetyl ether. The surfactants are added to enhance the wetness and solubilizing action of the microbicides.

International Publication No. WO 91/15122 discloses an anticorrosion bactericidal agent including peracetic acid, and a reaction product of fatty alcohol and phosphorus acid pentoxide and sodium hydroxide, or perfluoroalkylsulfonate potassium. It is described that the bactericidal agent kept the effectiveness as a bactericidal agent for at least seven days. The reaction product of fatty alcohol and phosphorus acid pentoxide and sodium hydroxide, or the perfluoroalkylsulfonate potassium is added in order to enhance the anticorrosion property.

U.S. Patent Nos. 4,051,058 and 4,051,059 disclose antibacterial agents including peracetic acid, and sulfonate- and sulfate-type cationic surfactants.

Japanese Patent No. 2523085 discloses a microbicide including peracetic acid as a main component. The microbicide is used to sterilize instruments used in surgery and dentistry. The microbicide is characterized by excluding a surfactant.

U.S. Patent No. 5,624,634 discloses a method for preparing a disinfectant composition for medical devices including metal components, in which a first aqueous solution including peracetic acid is mixed with a second aqueous solution including a corrosion inhibitor, a hydrogen peroxide stabilizer and/or a peracetic acid stabilizer. The disinfectant composition is prepared for the purpose of preventing the corrosion of medical devices including metal components. U.S. Patent No. 5,624,634 does not teach the use of a nonionic surfactant. In U.S. Patent No. 5,624,634, a problem how to stabilize the peracetic acid concentration of a peracetic acid practical solution is not recognized. For example, it is described that the disinfectant solutions were replaced daily with fresh solutions (column 5, lines 21 to 22).

U.S. Patent No. 5,720,983 and Japanese National Phase PCT Laid-open Publication No. 7-502988 also disclose disinfectant compositions for medical equipment including metal components, but do not teach the use of a nonionic surfactant.

U.S. Patent No. 5,489,706 discloses a method for stabilizing peracetic acid comprising a step of adding 0.1 to 5% of aliphatic alcohol ethoxylate. The aliphatic alcohol ethoxylate is introduced into a peracetic acid solution either during its manufacture or when it has been produced, thereby increasing the stability of peracetic acid (column 2, lines 31 to 33). U.S. Patent No. 5,489,706 does not teach a peracetic acid composition including phosphate.

U.S. Patent No. 5,545,374 discloses microbicidal compositions used at pH 6.0 or more. The microbicidal compositions include peracetic acid and a nonionic surfactant according to the general chemical formula $R\text{-}(OCH_2CH_2)_n\text{-}(OCH_2CH_2CH_3)_p\text{-}OH$ where R represents an alkyl group of at least 6 carbon atoms, and n and p each represent an integer. U.S. Patent No. 5,545,374 does not also disclose or teach a peracetic acid composition including phosphate. The microbicidal compositions are effective for *Candida albicans*, *Pseudomonas aeruginosa,* and *Staphylococcus aureus*.

EP 0873687 discloses compositions containing peracetic acid, acetic acid, hydrogen peroxide, amine oxide and, optionally, a non-ionic surfactant. These compositions are used for disinfecting medical and surgical materials and for disinfecting re-useable medical equipment such as endoscopes.

DISCLOSURE OF THE INVENTION

The above-described glutaraldehyde preparation solution (e.g., 2% of the practical solution prepared by adding a buffer) has a shelf life of one to two weeks. Comparing a bactericide/disinfectant including peracetic acid as an active component with the glutaraldehyde preparation solution , it is difficult to maintain the peracetic acid concentration of the practical solution obtained by diluting the peracetic acid preparation. Therefore, conventional peracetic acid solutions are usable only once, or if stored after preparation, have a shelf life of only 1 to 3 days. The practical solution needs to be prepared every time medical instruments are sterilized or disinfected. Thus, the peracetic acid solutions need to be frequently replaced with fresh solutions for long-term use, leading to an increase in cost.

The inventors have vigorously studied to solve the above-described problems with conventional peracetic acid preparations. As a result, a composition of the present invention is discovered in which the peracetic acid concentration of a practical solution obtained by diluting the composition can be stabilized, and a bactericidal/disinfectant effect can be kept for a long term.

The present invention relates to claim 1. The preparation is diluted to provide a practical solution including peracetic acid at a concentration capable of destroying bacterial spores and acid-fast bacteria while keeping such a concentration for at least seven days.

Preferably, the phosphate is selected from the group consisting of sodium orthophosphate, potassium orthophosphate, sodium pyrophosphate, potassium pyrophosphate, sodium polyphosphate, potassium polyphosphate, and combinations thereof.

Preferably, the concentration of peracetic acid is in the range of 1 to 10% w/w.

Preferably, a phosphate concentration of the practical solution is in the range of 0.01 to 2% w/w.

Preferably, a nonionic surfactant concentration of the practical solution is in the range of 0.01 to 0.5% w/w.

Preferably, the present invention provides Claim 10.

Preferably, the first reagent is an equilibrium composition including 5 to 7% w/w peracetic acid, 7 to 9% w/w hydrogen peroxide, 30 to 36% w/w acetic acid, and water.

Preferably, the first reagent includes a stabilizer.

Preferably, the stabilizer is selected from the group consisting of orthophosphoric acid and pyrophosphoric acid.

Preferably, the first reagent includes a metal ion sequestering agent or an anticorrosion agent.

Preferably, the second reagent includes a metal ion sequestering agent, an anticorrosion agent, or a pigment.

Preferably, the first reagent includes at least one phosphate or at least one surfactant.

Preferably, the second reagent is a powder material or a solid material.

Preferably, the first and second reagents are provided in a form of a mixture thereof.

BRIEF DESCRIPTION OF THE DRAWINGS

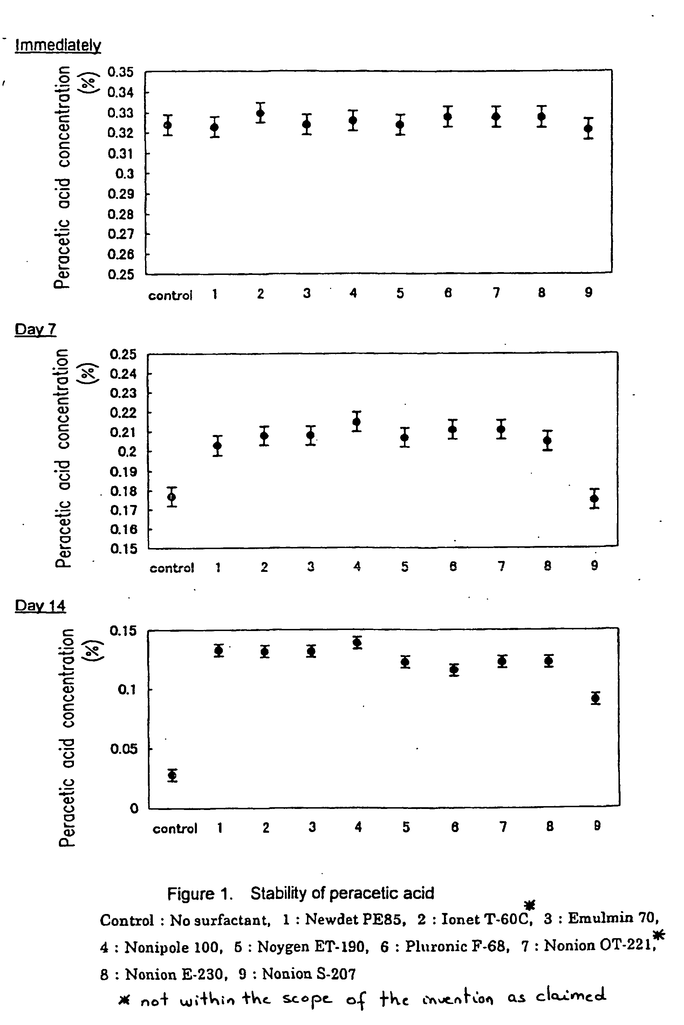

Figures **1** is a diagram showing a change in a peracetic acid concentration over time when various nonionic surfactants are added to a peracetic acid solution including sodium pyrophosphate which is in turn stored at room temperature.

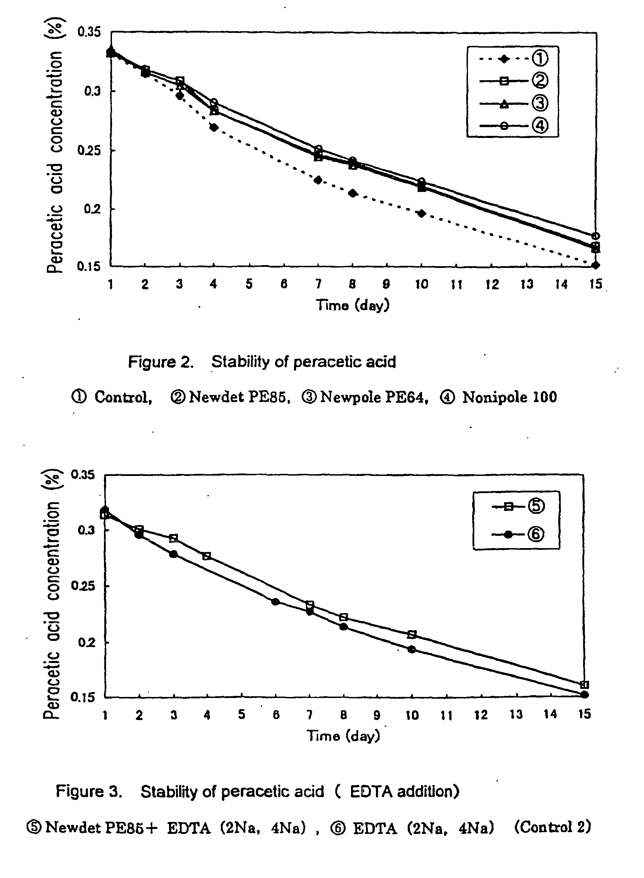

Figures **2** is a diagram showing a change in a peracetic acid concentration over time when various nonionic surfactants are added to a peracetic acid solution including dipotassium phosphate which is in turn stored at room temperature.

Figures **3** is a diagram showing a change in a peracetic acid concentration over time when a nonionic surfactant and a chelating agent are added to a peracetic acid solution including dipotassium phosphate which is in turn stored at room temperature.

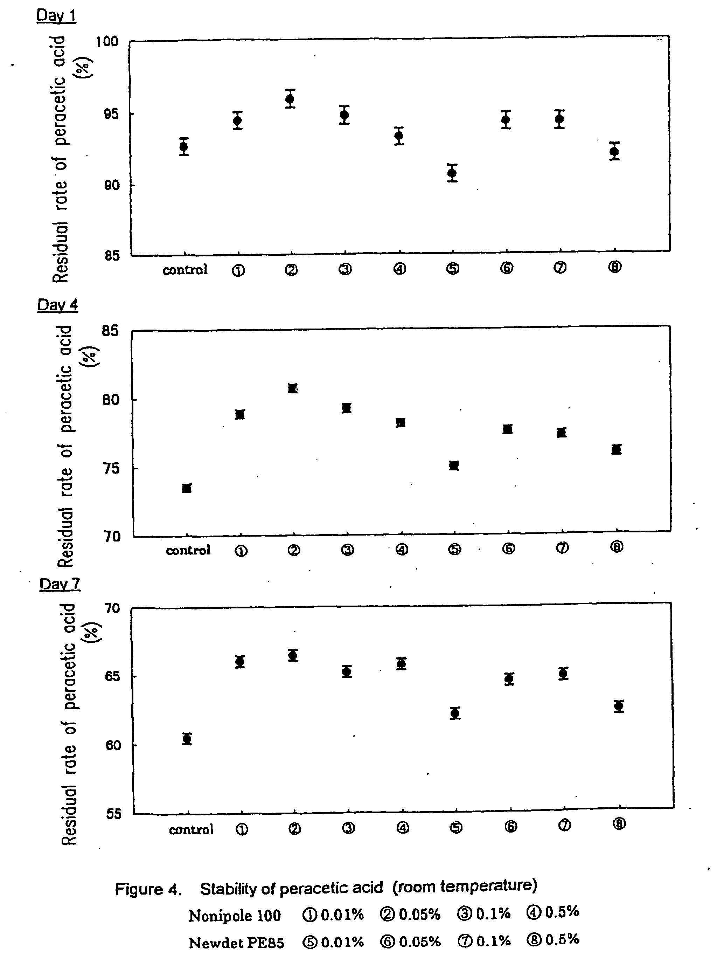

Figures **4** is a diagram showing a change in a peracetic acid concentration over time when a nonionic surfactant is added in various concentrations to a peracetic acid solution whose pH is adjusted to four with dipotassium phosphate, the solution being in turn stored at room temperature. The change is represented by a residual rate of peracetic acid.

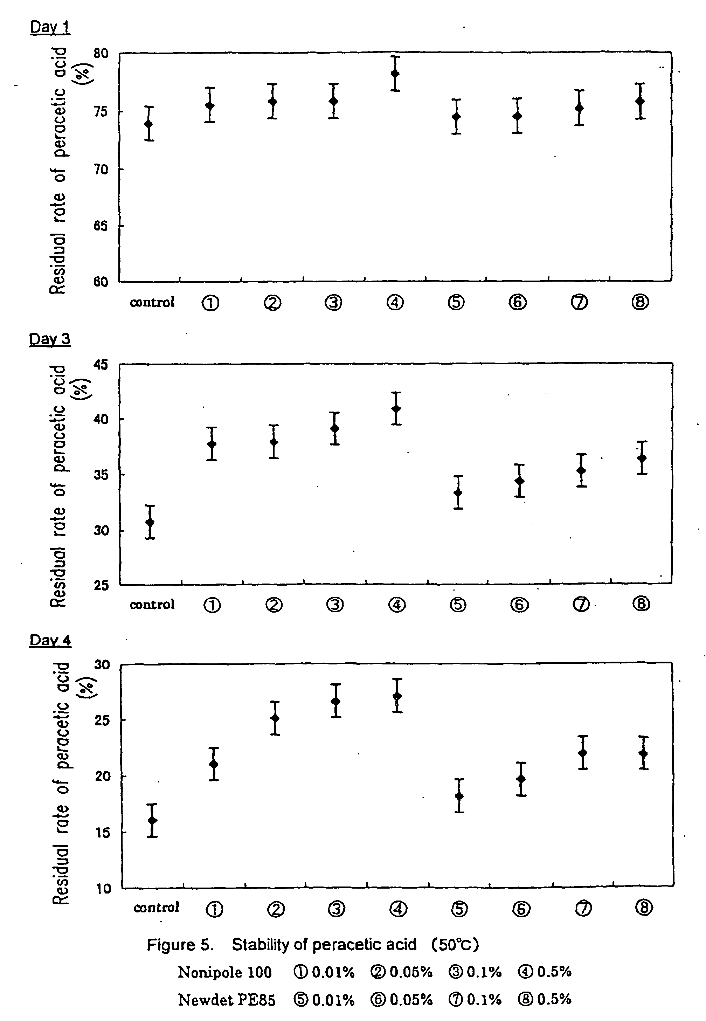

Figures **5** is a diagram showing a change in a peracetic acid concentration over time when a nonionic surfactant is added in various concentrations to a peracetic acid solution whose pH is adjusted to four with dipotassium phosphate, the solution being in turn stored at a high temperature (50°C). The change is represented by a residual rate of peracetic acid.

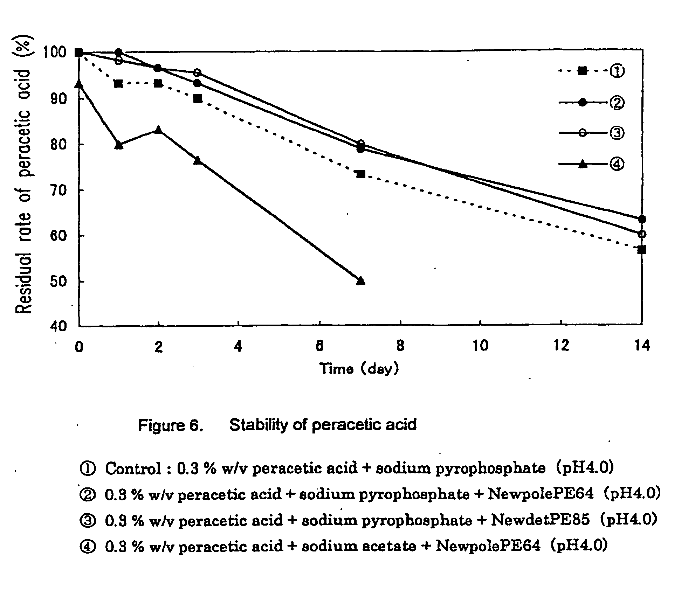

Figures **6** is a diagram showing a change in a peracetic acid concentration over time when a nonionic surfactant is added in various concentrations to a peracetic acid solution whose pH is adjusted to four with sodium pyrophosphate and sodium acetate, the solution being in turn stored at room temperature. The change is represented by a residual rate of peracetic acid.

BEST MODE FOR CARRYING OUT THE INVENTION

The present invention relates to a bacterial preparation including peracetic acid as a main component. The term "peracetic acid" refers to a compound having a molecular weight of 76.04 which usually exists as an equilibrium mixture of hydrogen peroxide, acetic acid, and water, or an organic solvent solution of acetic acid. A high-concentration bacterial preparation includes a concentration of around 40% of peracetic acid. A low-concentration bacterial preparation includes a concentration of 1% to 15% of peracetic acid. In Japan, preparations having a peracetic acid concentration of 4% and 6% are commercially available. The bactericidal preparation of the present invention including peracetic acid as a main component may be diluted to a peracetic acid concentration of 0.1% w/v to 1% w/v with water or preferably distilled water using a commercially available DIAPOWER (manufactured by Mitsubishi Gas Chemical Co., Inc) to prepare a practical solution. Peracetic acid smells badly, so that dilution is conducted in a facility equipped with a ventilator. Upon dilution, 0.01% w/w to 2% w/w of at least one phosphate and 0.01% w/w to 0.5% w/w of at least one nonionic surfactant are added to the peracetic acid solution followed by dissolution by stirring and mixing.

Preferably, the above-described phosphate is selected from the group consisting of sodium orthophosphate, potassium orthophosphate, sodium pyrophosphate, potassium pyrophosphate, sodium polyphosphate, potassium polyphosphate, and combinations thereof.

Preferably, the above-described nonionic surfactant is selected from the group consisting of a polyethylene/polypropylene block polymer type surfactant.

If the concentration of the peracetic acid is below 0.01% w/w, an immediate and sufficient bactericidal/disinfectant effect is not obtained. If the peracetic acid concentration exceeds 1% w/w, the smell and the irritation of eyes and skin are adversely significant.

If the concentration of the phosphate is below 0.01% w/w, the pH of the peracetic acid aqueous solution is smaller than or equal to 3. In this case, the chelating effect is lowered, and a corrosion property is adversely increased. If the concentration of the phosphate exceeds 2% w/w, the pH of the peracetic acid aqueous solution is greater than or equal to 5. In this case, the peracetic acid is unstable and a bactericidal effect is lowered.

If the concentration of the nonionic surfactant is below 0.01% w/w, peracetic acid is not sufficiently stabilized. If the concentration of the nonionic surfactant exceeds 0.5% w/w, the stabilization effect is not substantially improved, and foam does not disappear, which leads to practical inconvenience.

The bactericidal preparation including peracetic acid as a main component of the present invention is provided in a polyethylene container having a degassing stopper.

Further, the present invention provides a bactericidal preparation capable of destroying bacterial spores and acid-fast bacteria. The bactericidal preparation includes a first reagent including peracetic acid, hydrogen peroxide, acetic acid, and water, and a second reagent including at least one phosphate and at least one nonionic surfactant. The first and second reagents are mixed and diluted with water before use.

The bactericidal preparation of the present invention is preferably applied to a target object, such as medical instruments or medical equipment (e.g., an endoscope) after the target object is washed and rinsed with a neutral detergent or the like. The target object is immersed, for example, for 5 to 10 minutes in a practical solution prepared from the bactericidal preparation, exhibiting a bactericidal/disinfectant effect. The target object is available after being thoroughly rinsed with water.

The term "bacterial spore" as used herein refers to a resistant cell formed at the end of the growth phase of an aerobic bacillus *Bacillus subtillis*, an anaerobic bacillus of *Clostriduim* genus, or the like. The term "acid-fast bacterium" as used herein refers to an acid-fast stain positive bacterium well known to those skilled in the art, including bacteria of *Mycobacterium* genus.

The first reagent is an equilibrium mixture including 5 to 7% w/w of peracetic acid, 7 to 9% w/w of hydrogen peroxide, 30 to 36% w/w of acetic acid, and water. A commercially available low-concentration peracetic acid (e.g., brand name "DIAPOWER", manufactured by Mitsubishi Gas Chemical Co., Inc) may be used to obtain the first reagent. A stabilizer may be optionally added to the first reagent. The stabilizer is selected from the group consisting of orthophosphoric acid and pyrophosphoric acid. The stabilizer is typically added to the first reagent at a concentration of 0.2 to 1% w/w, followed by dissolution by stirring and mixing.

The first reagent may further optionally include a metal ion sequestering agent and an anticorrosion agent. The metal ion sequestering agent is typically added at a concentration of 0.1 to 2% w/w to the first reagent, respectively, followed by dissolution by stirring and mixing.

The second reagent may further optionally include a metal ion sequestering agent, an anticorrosion agent, and a pigment. The metal ion sequestering agent, the anticorrosion agent, and the pigment are each typically added at a concentration of 0.001 to 0.005% w/w to the second reagent, followed by dissolution by stirring and mixing.

The first reagent may further optionally include at least one phosphate and at least one nonionic surfactant.

The first reagent is typically provided in a polyethylene container having a degassing stopper. The second reagent is typically provided in a sealed polyethylene container. Alternatively, the first and second reagents are provided in a single polyethylene container having a degassing stopper in the form of a mixture.

The term "%" as used herein refers to "% w/w" unless it is otherwise described.

The concentration of peracetic acid and the bactericidal/disinfectant effect are determined by the following method.

Method for the determination of peracetic acid concentration

The peracetic acid concentration of a sample solution is measured according to a method described in Sully, B. D. and Williams, P. L., Analyst 1962; 87: 653-657.

A sample is precisely weighed to obtain one gram of the sample. The sample is added to 100 ml of a previously prepared 0.1 mol/L acetic acid solution kept at 5°C or less. To the resultant sample solution, 10 ml of a 15% w/v potassium iodide test solution is added, and simultaneously the measuring of time starts. Free iodine is measured by titration using 0.2 mol/L sodium thiosulfate until the blue color disappears where a starch test solution is used as an indicator. The titration is performed within about one to three minutes after the addition of the potassium iodide test solution. Thereafter, the titer ($X_1$ ml) and the time ($t_1$ sec) are measured the moment when the blue color reappears. After 2 to 4 minutes, the same process is repeated and the second titer ($X_2$ ml) and time ($t_2$ sec) are measured. Thereafter, three drops of 3.7 % w/v ammonium molybdenate test solution are added and titration is continued until an end point is stable for one minute. At this time point, the titer ($X_t$ ml) is measured. A titer at time zero, a peracetic acid concentration (%), and a hydrogen peroxide concentration (%) are calculated using the following formulas:

$$\text{Titer at time zero: } X_0 = X_1 - t_1(X_2 - X_1)/(t_2 - t_1)$$

$$\text{Peracetic acid concentration (\%)} = X_0 \times 0.2 \times f \times E/(10 \times W_1)$$

$$\text{Hydrogen peroxide concentration (\%)} = 0.2 \times f \times (X_1 - X_0) \times 17.01/(10 \times W_1)$$

where

$X_0$: titer at time zero
f: the factor of the 0.2 mol/L sodium thiosulfate solution
$W_1$: the amount of the sample (g)
E: the equivalent of peracetic acid (38.03)
P: the concentration of peracetic acid (%).

Method for testing bactericidal/disinfectant effect

The disinfectant effect of a peracetic acid solution is examined using the spores of *Bacillus subtilis*.

*Bacillus subtilis* (ATCC 6633) is cultivated on a liquid bouillon medium for 24 hours. Spores are prepared according to a method described in Sterlinin, J. M. and Mandelstem, J., Biochem. J. 1969; 113: 29. The spores are suspended in a sterilized distilled water. The suspension is heated at 85°C for 10 minutes to destroy vegetable cells. The resultant suspension is stored at 5°C. The spores are suspended in sterilized distilled water at $10^6$ to $10^7$ CFU/ml to obtain a spore sample suspension. The number of spores is calculated from the number of colonies obtained by pour plate culture in which test solutions serially diluted at 1/10 are poured onto bouillon agar media.

In the bactericidal/disinfectant testing, the number of spores is calculated from the number of colonies obtained by pour plate culture in which test solutions serially diluted at 1/10 are poured onto bouillon agar media.

(Examples)

Hereinafter, the present invention will be described by way of examples. The following examples are used to illustrate the present invention.

(Example 1)

A 0.33% peracetic acid aqueous solution (pH 4.0) was prepared which included 1% of sodium pyrophosphate and 0.05% of one nonionic surfactant shown in Table 1. The peracetic acid aqueous solution was put into a glass bin and stored at room temperature (25°C) for two weeks. During the storage, the peracetic acid aqueous solution was sampled over time so that the concentration of the peracetic acid aqueous solution was measured according to a method described in Sully, B. D. and Williams, P. L., Analyst 1962; 87: 653-657.

## Table 1 Nonionic surfactant additives

1. Newdet PE85 (polyoxyethylene/polyoxypropylnene block polymer, Sanyo Chemical Industries Co., Ltd.)

2.* Ionet T-60C (polyoxyethylene sorbitan fatty acid, Sanyo Chemical Industries Co., Ltd.)

3. Emulmin 70 (polyoxyethylene alkyl ether, Sanyo Chemical Industries Co., Ltd.)

4. Nonipole 100 (polyoxyethylene nonyl phenyl ether, Sanyo Chemical Industries Co., Ltd.)

5. Noygen ET-190 (polyethylene lauryl phenyl ether, Daiichi Kogyo Seiyaku, Co., Ltd.)

6. Pluronic F-68 (polyoxyethylene/polyoxypropylnene block polymer, Adeka)

7.* Nonion OT-221 (polyoxyethylene sorbitan monooleate, NOF Corporation)

8. Nonion S-230 (polyoxyethylene oleyl ether, NOF Corporation)

9. Nonion S-207 (polyoxyethylene stearyl ether, NOF Corporation)

*not within the scope of the invention as claimed*

The result of the measurement of the peracetic acid concentration of each sample is shown in Figure 1.

As shown in Figure 1, after one week, whereas the peracetic acid concentration was below 0.2% for a peracetic acid solution (control) including no nonionic surfactant, the peracetic acid concentration of 0.2% or more could be maintained for the peracetic acid solutions including the nonionic surfactants (1 to 9), except for the case where Nonion S-207 was added.

After two weeks, whereas the peracetic acid concentration was below 0.05% for the peracetic acid solution (control) including no nonionic surfactant, the peracetic acid concentration of 0.1% or more could be maintained for the peracetic acid solutions including the nonionic surfactants (1 to 9).

(Example 2)

Dipotassium hydrogen phosphate was added to a 0.3% peracetic acid solution so that the final concentration was 0.296% (pH 3.5). The resultant solution was divided into six solutions to which surfactants, reagents, and combinations thereof (① to ⑥ in Table 2) were added to obtain test solutions. The test solutions were stored at room temperature (25°C). The peracetic acid concentrations and the effect on bacterial spores of the test solutions were measured daily.

## <u>Table 2 Additives</u>

① No additive (Control 1)

② Newdet PE85 0.05%

③ Newpole PE64 0.05%

④ Nonipole 100 0.05%

⑤ Newdet PE85 0.05% + EDTA2Na 0.025%
                        + EDTA4Na 0.025%

⑥ EDTA2Na 0.025% + EDTA4Na 0.025% (Control 2)

The result of the measurement of the peracetic acid concentration of each sample is shown in Figures **2** and **3**.

As shown in Figures **2** and **3**, after **10** days of the room temperature storage, whereas the peracetic acid concentration was lowered up to about 0.2% for Control 1 and Control 2, the peracetic acid concentration of 0.2% or more could be maintained for any of the test solutions including surfactants, reagents, and combinations thereof.

Metal ions have adverse influence on the stability of peracetic acid. Therefore, a metal ion sequestering agent may be added in order to remove metal ions which are likely to be mixed into the solution in actual situations. However, the metal ion sequestering agent itself reduces the stability of the peracetic acid dilution. As shown in Figure 3, however, ⑤ is more stable than ⑥. It is recognized that peracetic acid can be stabilized even in the presence of the metal ion sequestering agent due to the addition of the nonionic surfactants.

The bactericidal/disinfectant effect on bacterial spores for each sample was measured. The results are shown in Table 3. To determine the bactericidal/disinfectant effect, the antimicrobial activity to *Bacillus subtilis* spores were examined for the samples including ② Newdet PE85 0.05%, ④ Nonipole 100 0.05%, and ⑤ Newdet PE85 0.05% + EDTA2Na 0.025% + EDTA4Na 0.025% of the samples described in Table 2.

Table 3

| Bactericidal/disinfectant effect of peracetic acid solutions | | | |
|---|---|---|---|
| Test solutions | ② | ④ | ⑤ |
| Action time | 5 min 10 min | 5 min 10 min | 5 min 10 min |
| Immediately after preparation | >6.01 >6.01 | >6.01 >6.01 | >6.01 >6.01 |
| Day 3 | >6.01 >6.01 | >6.01 >6.01 | >6.01 >6.01 |
| Day 7 | >6.01 >6.01 | >6.01 >6.01 | >6.01 >6.01 |
| Day 10 | >6.01 >6.01 | >6.01 >6.01 | >6.01 >6.01 |

The numerical values in the table represent the logarithmic values of the number of spores reduced by contacting spores with each sample solution for 5 minutes and 10 minutes.

As shown in Table 3, each sample solution has antimicrobial action such that *Bacillus subtilis* spores are reduced by $10^6$/ml or more even after 10 days storage.

(Example 3)

The following two preparations were prepared:

| First agent | |
|---|---|
| Peracetic acid | 6% |
| Hydrogen peroxide | 8% |
| Acetic acid | 32% |

(continued)

| First agent | |
|---|---|
| Phosphate type stabilizer | 0.2% |
| Water | balance |
| Second agent | |
| Dipotassium phosphate | 6% |
| Newdet PE85 (Sanyo Chemical Industries Co., Ltd.) | 1% |
| Disodium ethylene-diamine-teraacetate | 0.5% |
| Tetrasodium ethylene-diamine-teraacetate | 0.5% |
| Water | balance. |

The first and second agents were mixed in the same quantities, and diluted with a 10-fold capacity of water to obtain a test solution. The solution included about 0.33% of peracetic acid.

The test solution was used to sterilize and disinfect an endoscope using an automatic endoscope washer which is recently being utilized for that purpose.

The biopsy forceps of a flexible endoscope for use in the upper digestive tract (Olympus GIF type XQ240: manufactured by Olympus Optical Industries, Co., Ltd.) was washed and disinfected by an automatic endoscope washer (Olympus EW-30: manufactured by Olympus Optical Industries, Co., Ltd.) as follows. The automatic endoscope washer was set so that the biopsy forceps were washed once with tap water for 5 minutes and rinsed once with water, and thereafter washed and disinfected with the above-described test solution at 20°C for 10 minutes. The test solution was retained in the disinfectant liquid reservoir tank of the automatic endoscope washer. Six endoscopes were washed and disinfected a day (6 cycles per day). For the sixth endoscope, the inside of the biopsy forceps channel was contaminated with *Bacillus subtilis* spores in order to test the bactericidal/disinfectant effect of the test solution. 100 μl of *Bacillus subtilis* spore sample suspension including $10^7$ to $10^9$ CFU/ml of spores were applied to the inside of the biopsy forceps of the endoscope, and then dried.

The test solution was sampled from the disinfectant liquid reservoir tank of the washer after six cycles in each testing day. The peracetic acid concentration and the number of spores of the sample were measured by counting. Such a measurement was conducted everyday from the starting day of the testing to seventh day. The number of spores was measured as follows. A sterilized cotton wad which was impregnated with 5 ml of a sterilized recovering liquid was used to wipe the inside of the biopsy forceps channel so as to recover bacteria. This operation was repeated using 5 sterilized wads. The collection of the recovered bacteria was serially diluted at 1/10. The dilutions was subjected to pour plate culture using Triptosoy agar media. After 24 hour cultivation at 37°C, the number of surviving bacteria was measured by counting. The testing was conducted two times.

The results of the testing are shown in Table 4.

Table 4

| Bactericidal/disinfectant effect of peracetic acid solution | | | | | | | | |
|---|---|---|---|---|---|---|---|---|
| Test No.1 | Initial concentration | Day 1 | Day 2 | Day 3 | Day 4 | Day 5 | Day 6 | Day 7 |
| Peracetic acid (%) concentration (%) | 0.35 | 0.31 | 0.26 | 0.23 | 0.20 | 0.17 | 0.15 | 0.13 |
| Hydrogen peroxide concentration (%) | 0.44 | 0.31 | 0.34 | 0.34 | 0.31 | 0.31 | 0.26 | 0.24 |
| Residual rate of peracetic acid | 100% | 88.6% | 74.3% | 65.7% | 57,1% | 48.6% | 42.9% | 37.1% |
| Number of accumulated cycles | 0 | 6 | 12 | 18 | 24 | 30 | 36 | 42 |
| Logarithmic value of number of bacteria in sample bacteria suspension | | 8.57 | 8.68 | 8.15 | 8.38 | 8.30 | 8.32 | 8.53 |

Table 4   (continued)

| Bactericidal/disinfectant effect of peracetic acid solution | | | | | | | | |
|---|---|---|---|---|---|---|---|---|
| Test No.1 | Initial concentration | Day 1 | Day 2 | Day 3 | Day 4 | Day 5 | Day 6 | Day 7 |
| Number of recovered bacteria | | —a) | — | — | — | 1.40 | 1.40 | — |
| Logarithmic reduction value | | >7.87 | >7.98 | >7.45 | >7.68 | 6.90 | 6.92 | >7.83 |
| Test No.2 | Initial concentration | Day 1 | Day 2 | Day 3 | Day 4 | Day 5 | Day 6 | Day 7 |
| Peracetic acid (%) concentration (%) | 0.37 | 0.33 | 0.29 | 0.26 | 0.23 | 0.21 | 0.18 | 0.16 |
| Hydrogen peroxide concentration (%) | 0.44 | 0.43 | 0.40 | 0.38 | 0.33 | 0.30 | 0.27 | 0.27 |
| Residual rate of peracetic acid | 100% | 89.2 | 78.4% | 70.3% | 62.2% | 56.8% | 48.6% | 43.2% |
| Number of accumulated cycles | 0 | 6 | 12 | 18 | 24 | 30 | 36 | 42 |
| Logarithmic value of number of bacteria in sample bacteria suspension | | 7.83 | 8.18 | 8.46 | 8.26 | 8.46 | 8.45 | 8.52 |
| Number of recovered bacteria | | -- | — | — | — | 1.40 | 1.40 | 2.00 |
| Logarithmic reduction value | | >7.13 | >7.48 | >7.76 | 6.86 | 7.06 | >7.75 | 6.52 |

a) Less than the limitation of detection ($<5.0 \times 10^0$)

The peracetic acid concentration was reduced from 0.35% to 0.13% over time by seventh testing day. A dilution effect of rinsing water due to the structure of the device partially contributed to the reduction in the concentration.

The amount of spores was less than or equal to a limit of detection ($<5.0 \times 10°$) until the fourth day in the first testing and until the third day in the second testing. Several tens to 100/ml of spores were detected at the fifth and sixth day in the first testing and at the fourth, fifth, and seventh day in the second testing. Therefore, the spores were reduced to $1/10^6$ to $1/10^7$ in all days when testing was conducted. This shows that an effective peracetic acid concentration was maintained.

The above-described bactericidal/disinfectant effect on spore bearing bacteria shows that the test solution was sufficient for the washing and disinfection of an endoscope which had been used in an actual medical diagnosis. This is because spores are microorganism most resistant to bactricides/disinfectants, and it is not considered that endoscopes used in actual medical diagnoses and treatments were contaminated with a vast amount of spores used in the above-described examples.

(Example 4)

Two kinds of nonionic surfactants (Nonipole 100 and Newdet PE85) were added in concentrations of 0 to 0.5% to a 0.3% w/v peracetic acid aqueous solution which was in turn set to pH 4 with dipotassium hydrogen phosphate, thereby preparing test solutions. The test solutions were stored at room temperature (25°C) and at a high temperature (50°C). The concentration of peracetic acid was measured over time.

A change in the peracetic acid concentration in the case of the room temperature storage is shown in Figure **4**. A change in the peracetic acid concentration in the case of the high temperature storage is shown in Figure **5**.

When each test solution was stored at room temperature, the peracetic acid concentration of any of the test solutions was maintained at about 90% or more at the first day. At the fourth day, whereas the peracetic acid concentration of a control test solution including no surfactant was reduced to about 74% of the initial concentration, all of the test solutions including surfactants maintained about 75% or more of the initial concentration. After one week, whereas the peracetic acid concentration of a control test solution including no surfactant was reduced to about 61% of the initial concentration, all of the test solutions including surfactants maintained about 62% or more of the initial concentration.

When each test solution was stored at the high temperature, the peracetic acid concentration of all of the test solutions was maintained at about 74% or more at the first day. At the fourth day, whereas the peracetic acid concentration of a control test solution including no surfactant was reduced to about 31% of the initial concentration, all of the test solutions including surfactants maintained about 34% or more of the initial concentration. After one week, whereas the peracetic acid concentration of a control test solution including no surfactant was reduced to about 16% of the initial concentration, all of the test solutions including surfactants maintained about 17% or more of the initial concentration.

As described above, the peracetic acid concentration was maintained and stabilized by the addition of a nonionic surfactant either at room temperature or at a high temperature.

(Example 5)

Two kinds of nonionic surfactants (Newdet PE85100 and Newpole PE64 (polyoxyethylene/polyoxypropylene block polymer, Sanyo Chemical Industries Co., Ltd.)) were added in a concentration of 0.05% to a 0.3% w/v peracetic acid aqueous solution which was in turn set to pH 4 with sodium pyrophosphate or sodium acetate, thereby preparing test solutions. The test solutions were stored at room temperature (25°C). The concentration of peracetic acid was measured over time.

A change in the peracetic acid concentration over time is shown in Figure **6**.

When pH was set to **4** with sodium acetate, the peracetic acid concentration was reduced by about half even in the presence of a nonionic surfactant after seven days. When pH was set to 4 with pyrophosphate, about 80% of the peracetic acid concentration remained in the presence of a nonionic surfactant after seven days. About 75% of the peracetic acid concentration remained in the absence of a nonionic surfactant after seven days.

(Example 6)

The bactericidal effect of the peracetic acid preparation of the present invention was examined for *Mycobacterium tuberculosis* and atypical mycobacteria.

The following two preparations were prepared and used for testing:

| First agent | |
| --- | --- |
| Peracetic acid | 6% |
| Hydrogen peroxide | 8% |
| Acetic acid | 32% |
| Water | balance |
| Second agent | |
| Dipotassium phosphate | 6% |
| Newdet PE85 (Sanyo Chemical Industries Co., Ltd.) | 1% |
| Water | balance. |

The first and second agents were mixed in the same quantities, and diluted with sterilized distilled water to obtain peracetic acid practical solutions having respective concentrations of 0.3%, 0.2%, and 0.1%.

A commercially available glutaraldehyde preparation, Stelihide (manufactured by Maruishi Pharmaceutical Co., Ltd.) was used as a control. According to the manufacturer's instructions for the preparation, a buffer was added to the preparation to obtain a 2% glutaraldehyde solution which was used as a control of the test.

As subjects used for testing the bactericidal/disinfectant effect, the following *Mycobacterium tuberculosis* and atypical mycobacteria were used:

*Mycobacterium tuberculosis* H37Rv, *Mycobacterium avium* ATCC 15769, *Mycobacterium intracellurare* ATCC 13950, *Mycobacterium kansasii* ATCC 25414, *Mycobacterium tuberculosis* clinical isolate strain No. 1, and *Mycobacterium tuberculosis* clinical isolate strain No. 2.

The above-described sample bacteria were each cultivated on 1% Ogawa media (manufactured by Eiken Chemicals Co., Ltd.) (hereinafter referred to as "Ogawa media"). A platinum loop of the grown bacterial plaques was put into a test tube with a screw cap containing 5 sterilized glass beads each having a diameter of 5 mm and two drops of sterilized 10% Tween80. The test tube was stirred by shaking using an automatic mixer for 15 seconds. 4 ml of 7H9 bouillon (manufactured by Difco) was added to the test tube to obtain a homogenous suspension of cells of each sample bacetrium. The absorbance of the suspension was measured at a wavelength of 660 nm using a photometer (Mini photo 518, TAITEC). Thereafter, the suspension was diluted with sterilized water to adjust to 0.30. The resultant dilution is used as an inoculum liquid. The number of living bacteria in the inoculum liquid was evaluated as follows. The inoculum liquid was diluted at $1/10^2$, $1/10^3$, $1/10^4$, $1/10^5$, and $1/10^6$. 100 µl of each dilution was inoculated onto an Ogawa medium, and cultivated at 37°C. The number of grown colonies was measured by counting.

20 µl of the inoculum liquid was added and mixed with 500 µl of the above-described peracetic acid practical solution or 500 µl of a control glutaraldehyde solution, and allowed to stand at room temperature for a predetermined time (30 sec, 1 min, 2.5 min (3 min), 5 min, and 10 min), thereby contacting the preparation with the bacteria. 10 µl of the resultant mixture was inoculated to the 7H9 bouillon using a quantitative platinum loop (Bioloop, manufactured by ELKAY Co., Ltd.), and cultivated at 37°C. After four weeks, the presence or absence of grown bacteria was observed. A sample exhibiting the growth of bacteria was designated as growth positive (+), while a sample not exhibiting the growth of bacteria was designated as growth negative (-).

The results are shown in Table 5. As seen from Table 5, although a difference in sensitivity was recognized depending on the types or strains of anti-fact bacteria, all sample bacteria were destroyed within 30 seconds with the 0.3% peracetic acid solution. In contrast, when the 2% glutaraldehyde was used, some bacteria were not destroyed even after 10 minute exposure. Therefore, the bactericidal activity of the 2% glutaraldehyde was less than that of the 0.1% peracetic acid solution.

Thus, it was observed that the peracetic acid preparation of the present invention has a bactericidal/disinfectant effect on spore bearing bacteria and a bactericidal rate greater than those of glutaraldehyde, even in a relatively low concentration.

Table 5

| Bactericidal/disinfectant effect of peracetic acid solution on acid-fast bacteria | | | | | | | |
|---|---|---|---|---|---|---|---|
| | | Peracetic acid concentration 0.3% | | | | | |
| Strain | Control | 15sec. | 30sec. | 1min. | 2.5min. | 5min. | 10min. |
| *M.tuberculosis* H37Rv | + | -~+ | - | - | - | - | - |
| *M.avium* ATCC15769 | + | + | - | - | - | - | - |
| *M.intracellulare* ATCC13950 | + | -~+ | - | - | - | - | - |
| *M.kansasii* ATCC25414 | + | - | - | - | - | - | - |
| *M.tuberculosis* isolate strain No.1 | + | + | - | - | - | - | - |
| *M.tuberculosis* isolate strain No.2 | NT | NT | NT | NT | NT | NT | NT |
| | | Peracetic acid concentration 0.2% | | | | | |
| Strain | Control | 15sec. | 30sec. | 1min. | 2.5min. | 5min. | 10min. |
| *M.tuberculosis* H37Rv | + | + | -~+ | - | - | - | - |
| *M.avium* ATCC15769 | + | + | - | - | - | - | - |
| *M.intracellulare* ATCC13950 | + | + | - | - | - | - | - |
| *M.kansasii* ATCC25414 | + | - | - | - | - | - | - |
| *M.tuberculosis* isolate strain No.1 | + | + | + | - | - | - | - |
| *M.tuberculosis* isolate strain No.2 | + | + | + | - | - | - | - |
| | | Peracetic acid concentration 0.1% | | | | | |
| Strain | Control | 15sec. | 30sec. | 1min. | 2.5min. | 5min. | 10min. |
| *M.tuberculosis* H37Rv | + | + | + | -~+ | - | - | - |
| *M.avium* ATCC15769 | + | + | + | -~+ | - | - | - |
| *M.intracellulare* ATCC13950 | + | + | + | - | - | - | - |
| *M.kansasii* ATCC25414 | + | + | -~+ | - | - | - | - |
| *M.tuberculosis* isolate strain No.1 | + | + | + | - | - | - | - |
| *M.tuberculosis* isolate strain No.2 | + | + | + | - | - | - | - |

Table 5   (continued)

| Bactericidal/disinfectant effect of peracetic acid solution on acid-fast bacteria | | | | | | | |
|---|---|---|---|---|---|---|---|
| | | 2%glutaraldehyde | | | | | |
| Strain | Control | 15sec. | 30sec. | 1min | 2.5min. | 5min. | 10min. |
| *M.tuberculosis* H37Rv | + | + | + | -~+ | -~+ | -~+ | - |
| *M.avium* ATCC15769 | + | + | + | + | -~+ | -~+ | -~+ |
| *M.intracellulare* ATCC13950 | + | + | + | + | - | - | - |
| *M.kansasii* ATCC25414 | + | + | -~+ | - | - | - | - |
| *M.tuberculosis* isolate strain No.1 | + | + | + | + | - | - | - |
| *M.tuberculosis* isolate strain No.2 | NT | NT | NT | NT | NT | NT | NT |

INDUSTRIAL APPLICABILITY

The present invention provides a peracetic acid preparation used for sterilizing and disinfecting medical instruments, and medical equipment such as an endoscope, linen, or other goods. The peracetic acid preparation of the present invention stably maintains a concentration of peracetic acid capable of effectively destroying bacterial spores for at least seven days in a diluted practical solution. The preparation can substitute for a preparation including glutaraldehyde as an active component.

**Claims**

1. A bactericidal preparation including peracetic acid as a main component, comprising:

   peracetic acid;
   at least one phosphate; and
   at least one nonionic surfactant,

   wherein the bactericidal preparation is diluted with water to provide the practical solution;
   wherein the peracetic acid is a peracetic acid solution comprising an equilibrium concentration mixture of peracetic acid, hydrogen peroxide, acetic acid and water;
   wherein the at least one nonionic surfactant is selected from a polyoxyethylene/polyoxypropylene block polymer type surfactant; and
   wherein the at least one phosphate and the at least one nonionic surfactant are present in an amount sufficient to maintain the concentration of peracetic acid in the bactericidal practical solution.

2. A bactericidal preparation according to claim 1, wherein the bactericidal practical solution is capable of destroying a bacterial spore and an acid-fast bacterium.

3. A bactericidal preparation according to claim 2, wherein the concentration of peracetic acid in the bactericidal practical solution is sufficiently maintained to destroy a bacterial spore and an acid-fast bacterium for at least seven (7) days under room temperature.

4. A bactericidal preparation according to claim 3, wherein the phosphate is selected from the group consisting of sodium orthophosphate, potassium orthophosphate, sodium pyrophosphate, potassium pyrophosphate, sodium polyphosphate, potassium polyphosphate, and any combination thereof.

5. A bactericidal preparation according to claim 3, wherein the concentration of peracetic acid is in the range of 1 to 10% w/w.

6. A bactericidal preparation according to claim 3, wherein the concentration of peracetic acid in the bactericidal practical solution is in the range of 0.1 to 1% w/w.

7. A bactericidal preparation according to claim 3, wherein the phosphate concentration of the bactericidal practical solution is in the range of 0.01 to 2% w/w.

8. A bactericidal preparation according to claim 3, wherein the nonionic surfactant concentration of the bactericidal practical solution is in the range of 0.01 to 0.5% w/w.

9. A bactericidal preparation according to claim 7, wherein the pH of the bactericidal practical solution is in the range of pH 3 to 5.

10. A kit for providing a bactericidal practical solution, said kit comprising:

    a bactericidal preparation capable of destroying a bacterial spore and an acid-fast bacterium, comprising:

        a first reagent comprising a peracetic acid solution comprising an equilibrium concentration mixture of peracetic acid, hydrogen peroxide, acetic acid, and water; and
        a second reagent comprising at least one phosphate and at least one nonionic surfactant,

    wherein the at least one phosphate and the at least one nonionic surfactant are present in an amount sufficient to maintain the concentration of peracetic acid in the bactericidal practical solution;
    wherein the at least one nonionic surfactant is selected from the group consisting of a polyoxyethylene/polyoxypropylene block polymer type surfactant and a polyoxyethylene ether type surfactant;
    wherein the first and second reagents are provided separately; and
    wherein the first and second reagents are mixed and diluted with water before use.

11. The kit according to claim 10, wherein the first reagent is an equilibrium composition comprising 5 to 7% w/w peracetic acid, 7 to 9% w/w hydrogen peroxide, 30 to 36% w/w acetic acid, and water.

12. The kit according to claim 10, wherein the first reagent further comprises a stabilizer.

13. The kit according to claim 12, wherein the stabilizer is selected from the group consisting of orthophosphoric acid and pyrophosphoric acid.

14. The kit according to claim 10, wherein the first reagent further comprises a metal ion sequestering agent or an anticorrosion agent.

15. The kit according to claim 10, wherein the second reagent further comprises a metal ion sequestering agent, an anticorrosion agent, or a pigment.

16. Use of at least one phosphate and at least one nonionic surfactant to maintain the concentration of peracetic acid in a bactericidal practical solution, wherein the bactericidal practical solution is produced by diluting a bactericidal preparation with water;
    wherein the bactericidal preparation which includes peracetic acid as a main component, comprises
        peracetic acid;
        at least one phosphate; and
        at least one nonionic surfactant,
    wherein the peracetic acid is a peracetic acid solution comprising an equilibrium concentration mixture of peracetic acid, hydrogen peroxide, acetic acid and water;
    wherein the at least one phosphate and the at least one nonionic surfactant are present in an amount sufficient to maintain the concentration of peracetic acid in the bactericidal practical solution; and
    wherein the at least one nonionic surfactant is selected from a polyoxyethylene/polyoxypropylene block polymer type surfactant.

17. Use according to claim 16, wherein the bactericidal practical solution is capable of destroying a bacterial spore and an acid-fast bacterium.

18. Use according to claim 17, wherein the concentration of peracetic acid in the bactericidal practical solution is sufficiently maintained to destroy a bacterial spore and an acid-fast bacterium for at least seven (7) days under room temperature.

19. Use according to claim **18**, wherein the phosphate is selected from the group consisting of sodium orthophosphate, potassium orthophosphate, sodium pyrophosphate, potassium pyrophosphate, sodium polyphosphate, potassium polyphosphate, and any combination thereof.

**20.** Use according to daim 18, wherein the concentration of peracetic acid in the bactericidal practical solution is in the range of 0.1 to 1%, w/w.

**21.** Use according to claim 18, wherein the phosphate concentration of the bactericidal practical solution is in the range of 0.01 to 2% w/w.

**22.** Use according to claim 18, wherein the nonionic surfactant concentration of the bactericidal practical solution is in the range of 0.01 to 0.5% w/w.

**23.** Use according to claim 18, wherein the pH of the bactericidal practical solution is in the range of pH 3 to 5.

**24.** Use according to claim 16 wherein the phosphate is selected from dipotassium hydrogen phosphate or dipotassium phosphate.

**25.** A bactericidal preparation according to claim 1, wherein the phosphate is selected from dipotassium hydrogen phosphate or dipotassium phosphate.

**Patentansprüche**

**1.** Bakterizide Zubereitung, umfassend Peressigsäure als Hauptbestandteil, umfassend:

Peressigsäure;
mindestens ein Phosphat; und
mindestens ein nichtionisches Detergenz,

worin die bakterizide Zubereitung mit Wasser verdünnt wird, um die angewandte Lösung bereitzustellen;
worin die Peressigsäure eine Peressigsäurelösung darstellt, umfassend eine Gleichgewichtskonzentrationsmischung von Peressigsäure, Wasserstoffperoxid, Essigsäure und Wasser;
worin das mindestens eine nichtionische Detergenz ausgewählt ist unter einem Detergenz vom Typ des Polyoxyethylen/Polyoxypropylen-Blockpolymeren, und
worin das mindestens eine Phosphat und das mindestens eine nicht-ionische Detergenz in einer ausreichenden Menge vorhanden sind, um die Konzentration an Peressigsäure in den angewandten bakteriziden Lösung aufrecht zu erhalten.

**2.** Bakterizide Zubereitung nach Anspruch 1, worin die angewandte bakterizide Lösung eine Bakterienspore und ein säurebeständiges Bakterium zerstören kann.

**3.** Bakterizide Zubereitung nach Anspruch 2, worin die Konzentration an Peressigsäure in der angewandten bakteriziden Lösung für mindestens sieben (7) Tage unter Raumtemperatur ausreichend aufrechterhalten wird, um eine Bakterienspore und ein säurebeständiges Bakterium zu zerstören.

**4.** Bakterizide Zubereitung nach Anspruch 3, worin das Phosphat ausgewählt ist aus der Gruppe, bestehend aus Natriumorthophosphat, Kaliumorthophosphat, Natriumpyrophosphat, Kaliumpyrophosphat, Natriumpolyphosphat, Kaliumpolyphosphat und jeglicher Kombination davon.

**5.** Bakterizide Zubereitung nach Anspruch 3, worin die Konzentration an Peressigsäure im Bereich von 1 bis 10 % (Gewicht/Gewicht) liegt.

**6.** Bakterizide Zubereitung nach Anspruch 3, worin die Konzentration an Peressigsäure in der angewandten bakteriziden Lösung im Bereich von 0,1 bis 1 % (Gewicht/Gewicht) liegt.

**7.** Bakterizide Zubereitung nach Anspruch 3, worin die Phosphatkonzentration der angewandten bakteriziden Lösung im Bereich von 0,01 bis 2 % (Gewicht/Gewicht) liegt.

**8.** Bakterizide Zubereitung nach Anspruch 3, worin die Konzentration an nichtionischem Detergenz der angewandten bakteriziden Lösung im Bereich von 0,01 bis 0,5 % (Gewicht/Gewicht) liegt.

9. Bakterizide Zubereitung nach Anspruch 7, worin der pH-Wert der angewandten bakteriziden Lösung im Bereich von pH 3 bis 5 liegt.

10. Kit zur Bereitstellung einer angewandten bakteriziden Lösung, der Kit umfassend:

   eine bakterizide Zubereitung, welche eine Bakterienspore und säurebeständiges Bakterium zerstören kann, umfassend:

   ein erstes Reagenz, umfassend eine Peressigsäurelösung, umfassend eine Gleichgewichtskonzentrationsmischung von Peressigsäure, Wasserstoffperoxid, Essigsäure und Wasser; und
   ein zweites Reagenz, umfassend mindestens ein Phosphat und mindestens ein nichtionisches Detergenz,

   worin das mindestens eine Phosphat und das mindestens eine nichtionische Detergenz in ausreichender Menge vorhanden sind, um die Konzentration an Peressigsäure in der angewandten bakteriziden Lösung aufrecht zu erhalten;
   worin das mindestens eine nichtionische Detergenz ausgewählt ist aus der Gruppe, bestehend aus eine Detergenz vom Typ des Polyoxyethylen/Polyoxypropylen-Blockpolymeren und einem Detergenz vom Typ des Polyoxyethylenethers;
   worin das erste und das zweite Reagenz getrennt bereitgestellt werden und
   worin das erste und das zweite Reagenz vor der Verwendung gemischt und mit Wasser verdünnt werden.

11. Kit nach Anspruch 10, worin das erste Reagenz eine Gleichgewichtszusammensetzung ist, umfassend 5 bis 7 % (Gewicht/Gewicht) Peressigsäure, 7 bis 9 % (Gewicht/Gewicht) Wasserstoffperoxid, 30 bis 36 % (Gewicht/Gewicht) Essigsäure und Wasser.

12. Kit nach Anspruch 10, worin das erste Reagenz zusätzlich einen Stabilisator umfasst.

13. Kit nach Anspruch 12, worin der Stabilisator ausgewählt ist aus der Gruppe, bestehend aus Orthophosphorsäure und Pyrophosphorsäure.

14. Kit nach Anspruch 10, worin das erste Reagenz zusätzlich ein Sequestriermittel für Metallionen oder einen Korrosionsinhibitor umfasst.

15. Kit nach Anspruch 10, worin das zweite Reagenz zusätzlich ein Sequestriermittel für Metallionen, einen Korrosionsinhibitor oder ein Pigment umfasst.

16. Verwendung mindestens eines Polyphosphats und mindestens eines nichtionischen Detergenz zum Aufrechterhalten der Konzentration von Peressigsäure in einer angewandten bakteriziden Lösung, worin die bakterizide Lösung hergestellt wird durch Verdünnen einer bakteriziden Zubereitung mit Wasser;
worin die bakterizide Zubereitung, die Peressigsäure als Hauptbestandteil enthält, umfasst:

   Peressgisäure;
   mindestens ein Phosphat; und
   mindestens ein nichtionisches Detergenz,

worin die Peressigsäure eine Peressigsäurelösung darstellt, umfassend eine Gleichgewichtskonzentrationsmischung von Peressigsäure, Wasserstoffperoxiod, Essigsäure und Wasser;
worin das mindestens eine Phosphat und das mindestens eine nichtionische Detergenz in ausreichender Menge vorhanden sind, um die Konzentration an Peressigsäure in der angewandten bakteriziden Lösung aufrecht zu erhalten, und
worin das mindestens eine nichtionische Detergenz ausgewählt ist unter einem Detergenz vom Typ des Polyoxyethylen/Polyoxypropylen-Blockpolymeren.

17. Verwendung nach Anspruch 16, worin die angewandte bakterizide Lösung eine Bakterienspore und ein säurebeständiges Bakterium zerstören kann.

**18.** Verwendung nach Anspruch 17, worin die Konzentration an Peressigsäure in der angewandten bakteriziden Lösung für mindestens sieben (7) Tage unter Raumtemperatur ausreichend erhalten wird, um eine Bakterienspore und ein säurebeständiges Bakterium zu zerstören.

**19.** Verwendung nach Anspruch 18, worin das Phosphat ausgewählt ist aus der Gruppe, bestehend aus Natriumorthophosphat, Kaliumorthophosphat, Natriumpyrophosphat, Kaliumpyrophosphat, Natriumpolyphosphat, Kaliumpolyphosphat und jeglicher Kombination davon.

**20.** Verwendung nach Anspruch 18, worin die Konzentration an Peressigsäure in der angewandten bakteriziden Lösung im Bereich von 0,1 bis 1 % (Gewicht/Gewicht) liegt.

**21.** Verwendung nach Anspruch 18, worin die Phosphatkonzentration der angewandten bakteriziden Lösung im Bereich von 0,01 bis 2 % (Gewicht/Gewicht) liegt.

**22.** Verwendung nach Anspruch 18, worin die Konzentration an nichtionischem Detergenz der angewandten bakteriziden Lösung im Bereich 0,01 bis 0,5 % (Gewicht/Gewicht) liegt.

**23.** Verwendung nach Anspruch **18**, worin der pH-Wert der angewandten bakteriziden Lösung im Bereich von pH 3 bis 5 liegt.

**24.** Verwendung nach Anspruch 16, worin das Phosphat ausgewählt ist unter Dikaliumhydrogenphosphat oder Dikaliumphosphat.

**25.** Bakterizide Zubereitung nach Anspruch 1, worin das Phosphat ausgewählt ist unter Dikaliumhydrogenphosphat oder Dikaliumphosphat.

**Revendications**

**1.** Préparation bactéricide comprenant de l'acide peracétique comme composant principal, comportant :

de l'acide peracétique;
au moins un phosphate; et
au moins un agent tensioactif non ionique,

dans laquelle la préparation bactéricide est diluée avec de l'eau pour fournir la solution pratique;
dans laquelle l'acide peracétique est une solution d'acide peracétique comprenant un mélange de concentrations à l'équilibre d'acide peracétique, de peroxyde d'hydrogène, d'acide acétique et d'eau;
dans laquelle le au moins un agent tensioactif est choisi dans un tensioactif de type polymère à séquences de polyoxyéthylène/polyoxypropylène; et
dans laquelle le au moins un phosphate et le au moins un agent tensioactif non ionique sont présents en quantité suffisante pour maintenir la concentration en acide peracétique dans la solution bactéricide pratique.

**2.** Préparation bactéricide selon la revendication 1, dans laquelle la solution bactéricide pratique est capable de détruire une spore bactérienne et une bactérie résistant aux acides.

**3.** Préparation bactéricide selon la revendication 2, dans laquelle la concentration en acide peracétique dans la solution bactéricide pratique est maintenue suffisamment pour détruire une spore bactérienne et une bactérie résistant aux acides pendant au moins sept (7) jours à température ambiante.

**4.** Préparation bactéricide selon la revendication 3, dans laquelle le phosphate est choisi dans le groupe constitué de l'orthophosphate de sodium, de l'orthophosphate de potassium, du pyrophosphate de sodium, du pyrophosphate de potassium, du polyphosphate de sodium, du polyphosphate de potassium et d'une quelconque de leurs combinaisons.

**5.** Préparation bactéricide selon la revendication 3, dans laquelle la concentration en acide peracétique se situe dans la plage de 1 à 10% en poids/poids.

**6.** Préparation bactéricide selon la revendication 3, dans laquelle la concentration en acide peracétique dans la solution bactéricide pratique se situe dans la plage de 0,1 à 1% en poids/poids.

**7.** Préparation bactéricide selon la revendication 3, dans laquelle la concentration en phosphate de la solution bactéricide pratique se situe dans la plage de 0,01 à 2% en poids/poids.

**8.** Préparation bactéricide selon la revendication 3, dans laquelle la concentration en agent tensioactif non ionique de la solution bactéricide pratique se situe dans la plage de 0,01 à 0,5% en poids/poids.

**9.** Préparation bactéricide selon la revendication 7, dans laquelle le pH de la solution bactéricide pratique se situe dans la plage de pH de 3 à 5.

**10.** Kit pour fournir une solution bactéricide pratique, ledit kit comprenant :

une préparation bactéricide capable de détruire une spore bactérienne et une bactérie résistant aux acides, comprenant :

un premier réactif comprenant une solution d'acide peracétique comprenant un mélange de concentrations à l'équilibre d'acide peracétique, de peroxyde d'hydrogène, d'acide acétique et d'eau ; et
un second réactif comprenant au moins un phosphate et au moins un agent tensioactif non ionique,

dans lequel le au moins un phosphate et le au moins un agent tensioactif non ionique sont présents en quantité suffisante pour maintenir la concentration en acide peracétique dans la solution bactéricide pratique;
dans lequel le au moins un agent tensioactif non ionique est choisi dans le groupe constitué d'un agent tensioactif de type polymère à séquences de polyoxyéthylène/polyoxypropylène et d'un agent tensioactif de type éther de polyoxyéthylène;
dans lequel les premier et second réactifs sont fournis séparément; et
dans lequel les premier et second réactifs sont mélangés et dilués avec de l'eau avant utilisation.

**11.** Kit selon la revendication 10, dans lequel le premier réactif est une composition à l'équilibre comprenant 5 à 7% en poids/poids d'acide peracétique, 7 à 9% en poids/poids de peroxyde d'hydrogène, 30 à 36% en poids/poids d'acide acétique et de l'eau.

**12.** Kit selon la revendication 10, dans lequel le premier réactif comprend en outre un stabilisateur.

**13.** Kit selon la revendication 12, dans lequel le stabilisateur est choisi dans le groupe constitué de l'acide orthophosphorique et de l'acide pyrophosphorique.

**14.** Kit selon la revendication 10, dans lequel le premier réactif comprend en outre un agent séquestrant d'ions métalliques ou un agent anticorrosion.

**15.** Kit selon la revendication 10, dans lequel le second réactif comprend en outre un agent séquestrant d'ions métalliques, un agent anticorrosion ou un pigment.

**16.** Utilisation d'au moins un phosphate et d'au moins un agent tensioactif non ionique pour maintenir la concentration en acide peracétique dans une solution bactéricide pratique, dans lequel la solution bactéricide pratique est produite en diluant une préparation bactéricide avec de l'eau;
dans laquelle la préparation bactéricide qui comprend de l'acide peracétique comme composant principal, comporte :

de l'acide peracétique;
au moins un phosphate; et
au moins un agent tensioactif non ionique,

dans laquelle l'acide peracétique est une solution d'acide peracétique comprenant un mélange de concentrations à l'équilibre d'acide peracétique, de peroxyde d'hydrogène, d'acide acétique et d'eau;
dans laquelle le au moins un phosphate et le au moins un agent tensioactif non ionique sont présents en quantité suffisante pour maintenir la concentration en acide peracétique dans la solution bactéricide pratique; et

dans laquelle le au moins un agent tensioactif est choisi dans un agent tensioactif de type polymère à séquences de polyoxyéthylène/polyoxypropylène.

17. Utilisation selon la revendication 16, dans laquelle la solution bactéricide pratique est capable de détruire une spore bactérienne et une bactérie résistant aux acides.

18. Utilisation selon la revendication 17, dans laquelle la concentration en acide peracétique dans la solution bactéricide pratique est maintenue suffisamment pour détruire une spore bactérienne et une bactérie résistant aux acides pendant au moins sept (7) jours à température ambiante.

19. Utilisation selon la revendication 18, dans laquelle le phosphate est choisi dans le groupe constitué de l'orthophosphate de sodium, de l'orthophosphate de potassium, du pyrophosphate de sodium, du pyrophosphate de potassium, du polyphosphate de sodium, du polyphosphate de potassium et de l'une quelconque de leurs combinaisons.

20. Utilisation selon la revendication 18, dans laquelle la concentration en acide peracétique dans la solution bactéricide pratique se situe dans la plage de 0,1 à 1% en poids/poids.

21. Utilisation selon la revendication 18, dans laquelle la concentration en phosphate dans la solution bactéricide pratique se situe dans la plage de 0,01 à 2% en poids/poids.

22. Utilisation selon la revendication 18, dans laquelle la concentration en agent tensioactif non ionique dans la solution bactéricide pratique se situe dans la plage de 0,01 à 0,5% en poids/poids.

23. Utilisation selon la revendication 18, dans laquelle le pH de la solution bactéricide pratique se situe dans la plage de pH de 3 à 5.

24. Utilisation selon la revendication 16, dans laquelle le phosphate est choisi parmi l'hydrogénophosphate dipotassique ou le phosphate dipotassique.

25. Préparation bactéricide selon la revendication 1, dans laquelle le phosphate est choisi parmi l'hydrogénophosphate dipotassique et le phosphate dipotassique.

__Immediately__

__Day 7__

__Day 14__

Figure 1.    Stability of peracetic acid

Control : No surfactant,  1 : Newdet PE85,  2 : Ionet T-60C,  3 : Emulmin 70,
4 : Nonipole 100,  5 : Noygen ET-190,  6 : Pluronic F-68,  7 : Nonion OT-221,
8 : Nonion E-230,  9 : Nonion S-207

*✳ not within the scope of the invention as claimed*

Figure 2.   Stability of peracetic acid

① Control,   ② Newdet PE85,   ③ Newpole PE64,   ④ Nonipole 100

Figure 3.   Stability of peracetic acid  ( EDTA addition)

⑤ Newdet PE85+ EDTA (2Na, 4Na) , ⑥ EDTA (2Na, 4Na)   (Control 2)

Day 1

Day 4

Day 7

Figure 4. Stability of peracetic acid (room temperature)

Nonipole 100   ① 0.01%   ② 0.05%   ③ 0.1%   ④ 0.5%

Newdet PE85   ⑤ 0.01%   ⑥ 0.05%   ⑦ 0.1%   ⑧ 0.5%

**Day 1**

**Day 3**

**Day 4**

Figure 5.   Stability of peracetic acid   (50℃)

Nonipole 100    ① 0.01%   ② 0.05%   ③ 0.1%   ④ 0.5%

Newdet PE85    ⑤ 0.01%   ⑥ 0.05%   ⑦ 0.1%   ⑧ 0.5%

Figure 6.   Stability of peracetic acid

① Control : 0.3 % w/v peracetic acid + sodium pyrophosphate (pH4.0)

② 0.3 % w/v peracetic acid + sodium pyrophosphate + NewpolePE64 (pH4.0)

③ 0.3 % w/v peracetic acid + sodium pyrophosphate + NewdetPE85 (pH4.0)

④ 0.3 % w/v peracetic acid + sodium acetate + NewpolePE64 (pH4.0)